(19) Europäisches Patentamt
European Patent Office
Office européen des brevets

(11) Numéro de publication: **0 293 294 A2**

(12) **DEMANDE DE BREVET EUROPEEN**

(21) Numéro de dépôt: **88401269.1**

(22) Date de dépôt: **25.05.88**

(51) Int. Cl.[4]: **C 07 D 487/04**
A 61 K 31/505
//(C07D487/04,239:00,235:00)

(30) Priorité: **27.05.87 GB 8712466**

(43) Date de publication de la demande:
**30.11.88 Bulletin 88/48**

(84) Etats contractants désignés:
**AT BE CH DE ES FR GR IT LI LU NL SE**

(71) Demandeur: **ROUSSEL-UCLAF**
**35, boulevard des Invalides**
**F-75007 Paris (FR)**

(72) Inventeur: **Gardner, Colin Robert**
**Foxlea-Hermitage Road Cold Ash**
**Newbury Berks (GB)**

**Hedgecock Charles John Robert**
**186 High Street**
**Wootton Bassett Wiltshire SN4 7BZ (GB)**

**Jones, Stuart Donald**
**1 Walnut Tree Gardens Lydiard Millicent**
**Swindon Wiltshire SN5 9LH (GB)**

(74) Mandataire: **Vieillefosse, Jean-Claude et al**
**Département des Brevets ROUSSEL UCLAF B.P no 9**
**F-93230 Romainville (FR)**

(54) **Nouvelles imidazo /1,2-a/ pyrimidines et leurs sels, leur procédé et les intermédiaires de préparation, l'application à titre de médicaments et les compositions les renfermant.**

(57) L'invention concerne de nouvelles imidazo [1,2-a] pyrimidines répondant à la formule :

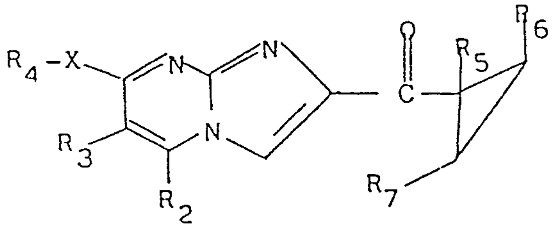

(I)

dans laquelle $R_2$ et $R_3$ représentent chacun hydrogène, alcoyle ($C_1$-$C_8$) éventuellement substitué par un cycloalcoyle ($C_3$-$C_7$), alcényle ($C_2$-$C_5$), cycloalcoyle ($C_3$-$C_7$), aralcoyle ($C_7$-$C_{15}$) ou $R_2$ et $R_3$ forment un alcoylène, chacun des radicaux ci-dessus pouvant être substitué par un ou plusieurs alcoyles ($C_1$-$C_5$), X est oxygène ou soufre, $R_4$ représente alcoyle ($C_1$-$C_5$), $R_5$ et $R_6$ représentent chacun hydrogène ou alcoyle ($C_1$-$C_5$), $R_7$ représente hydrogène, alcoyle ($C_1$-$C_5$), phényle, halogène, COOH, COOalcoyle ($C_2$-$C_5$), CN, amido, mono ou dialcoylamido avec alcoyle ($C_1$-$C_5$), étant que l'un au moins de $R_5$, $R_6$ et $R_7$ est différent de hydrogène,
ainsi que leurs sels, leur procédé de préparation, leur application comme médicaments et les compositions les renfermant.

EP 0 293 294 A2

## Description

### Nouvelles imidazo [1,2-a] pyrimidines et leurs sels, leur procédé et les intermédiaires de préparation, l'application à titre de médicaments et les compositions les renfermant.

La présente invention concerne de nouvelles imidazo [1,2-a] pyrimidines et leurs sels, ainsi que le procédé et les intermédiaires de préparation, l'application à titre de médicaments de ces nouveaux produits et les compositions les renfermant.

L'invention a pour objet de nouvelles imidazo [1,2-a] pyrimidines répondant à la formule (I) :

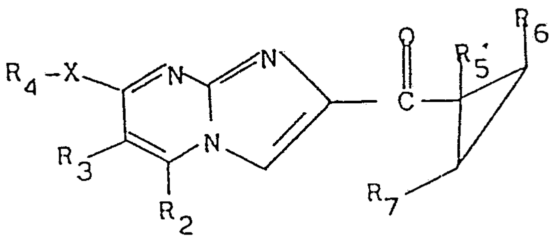

(I)

dans laquelle $R_2$ et $R_3$, identiques ou différents, représentent un atome d'hydrogène, un radical alcoyle linéaire renfermant de 1 à 8 atomes de carbone éventuellement substitué par un radical cycloalcoyle renfermant de 3 à 7 atomes de carbone, un radical alcényle linéaire renfermant de 2 à 5 atomes de carbone, un radical cycloalcoyle renfermant de 3 à 7 atomes de carbone, un radical aralcoyle renfermant de 7 à 15 atomes de carbone ou $R_2$ et $R_3$ forment ensemble un radical alcoylène renfermant de 3 à 5 atomes de carbone, chacun des radicaux ci-dessus pouvant être substitué par un ou plusieurs radicaux alcoyles renfermant de 1 à 5 atomes de carbone,
X représente un atome d'oxygène ou de soufre,
$R_4$ représente un radical alcoyle linéaire renfermant de 1 à 5 atomes de carbone,
$R_5$ représente un atome d'hydrogène ou un radical alcoyle renfermant de 1 à 5 atomes de carbone,
$R_6$ représente un atome d'hydrogène ou un radical alcoyle renfermant de 1 à 5 atomes de carbone,
$R_7$ représente un atome d'hydrogène, un radical alcoyle renfermant de 1 à 5 atomes de carbone, un radical phényle, un atome d'halogène, un radical hydroxycarbonyle, un radical alkoxycarbonyle renfermant de 2 à 5 atomes de carbone, un radical cyano, un radical amido, un radical mono ou dialcoylamido dont les radicaux alcoyles renferment de 1 à 5 atomes de carbone,
étant entendu que l'un au moins des radicaux $R_5$, $R_6$ et $R_7$ ne représentent pas un atome d'hydrogène,
ainsi que leurs sels d'addition avec les acides minéraux ou organiques.

Dans la formule générale (I) et dans ce qui suit :
- par un radical alcoyle renfermant de 1 à 8 atomes de carbone, on entend, de préférence, ceux renfermant de 1 à 5 atomes de carbone, plus particulièrement un radical méthyle, éthyle, propyle, butyle ou pentyle ou lorsque le radical alcoyle est substitué par un radical alcoyle, un radical isopropyle, isobutyle ou terbutyle ;
- par un radical alcényle, on entend, de préférence, un radical vinyle, allyle, but-3-ényle ou lorsque le radical alcényle est substitué par un radical alcoyle, un radical isopropényle ;
- par un radical cycloalcoyle, on entend, de préférence, un radical cyclopropyle, cyclobutyle, cyclopentyle, cyclohexyle ou cycloheptyle ;
- par un radical aralcoyle, on entend, de préférence, un radical benzyle ou phénéthyle ;
- par atome d'halogène, on entend, de préférence, un atome de fluor, de chlore ou de brome ;
- par radical alcoxycarbonyle renfermant de 2 à 5 atomes de carbone, on entend, de préférence, un radical méthoxycarbonyle, éthoxycarbonyle ou propoxycarbonyle ;
- par radical mono ou dialcoylamido dont les radicaux alcoyles renfermant de 1 à 5 atomes de carbone, on entend, de préférence, un radical monométhylamido, diméthylamido, monoéthylamido, diéthylamido, monopropylamido ou dipropylamido.

Les sels d'addition avec les acides minéraux ou organiques peuvent être par exemple, les sels formés avec les acides chlorhydrique, bromhydrique, iodhydrique, nitrique, sulfurique, phosphorique, propionique, formique, benzoïque, maléique, fumarique, succinique, tartrique, citrique, oxalique, glyoxylique, aspartique, alcanesulfoniques tels que l'acide méthanesulfonique et arylsulfoniques tels que l'acide benzènesulfonique.

Parmi les produits, objet de l'invention, on peut citer notamment les dérivés répondant à la formule (I) ci-dessus, ainsi que leurs sels, caractérisés en ce que dans ladite formule (I),
$R_2$, $R_3$, $R_4$ et X ont la signification déjà indiquée,
$R_5$ représente un atome d'hydrogène ou un radical méthyle,
$R_6$ représente un atome d'hydrogène ou un radical méthyle,
$R_7$ représente un atome d'hydrogène, un atome de chlore, un radical méthyle, un radical éthoxycarbonyle, un radical cyano, un radical amido, méthylamido ou diméthylamido,
étant entendu que l'un au moins des radicaux $R_5$, $R_6$ et $R_7$ ne représente pas un atome d'hydrogène.

Parmi ces derniers, on peut citer les dérivés répondant à la formule (I) ci-dessus ainsi que leurs sels, caractérisés en ce que dans ladite formule (I) :
$R_2$ et $R_3$, identiques ou différents, représentent un atome d'hydrogène, un radical méthyle ou éthyle, ou $R_2$ et $R_3$ forment ensemble un radical butylène,
X représente un atome d'oxygène ou de soufre,
$R_4$ représente un radical méthyle,
$R_5$, $R_6$ et $R_7$ ont la signification déjà indiquée.

Parmi les produits, objet de l'invention, on retient tout particulièrement ceux dont les noms suivent :
- la trans (6,7,8,9-tétrahydro-5-méthoxyimidazo [1,2-a] quinazolin-2-yl 2-chlorocyclopropylméthanone,
- le trans 2-(6-éthyl 7-méthoxy 5-méthylimidazo [1,2-a] pyrimidin-2-oyl) cyclopropane carboxylate d'éthyle,
- le trans 2-(6,7,8,9-tétrahydro-5-méthoxyimidazo [1,2-a] quinazolin-2-oyl) cyclopropane carbonitrile,
- la trans (6-éthyl 7-méthoxy 5-méthylimidazo [1,2-a] pyrimidin-2-yl) 2-méthyl cyclopropylméthanone,
- la (6,7,8,9-tétrahydro 5-méthoxyimidazo [1,2-a] quinazolin-2-yl) 1-méthyl cyclopropylméthanone, ainsi que leurs sels.

L'invention a également pour objet un procédé de préparation des nouvelles imidazo [1,2-a] pyrimidines telles que définies par la formule (I) ci-dessus dans laquelle $R_2$, $R_3$, $R_4$, $R_6$ et X ont la signification déjà indiquée, $R_5$ représente un atome d'hydrogène et $R_7$ représente un atome d'hydrogène ou un radical alcoyle renfermant de 1 à 5 atomes de carbone, ainsi que de leurs sels, caractérisé en ce que l'on fait réagir un produit de formule (III) :

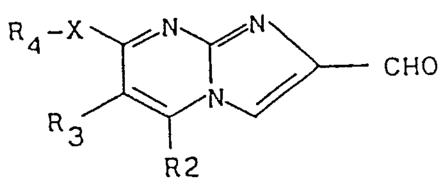

(III)

dans laquelle $R_2$, $R_3$, $R_4$ et X ont la signification déjà indiquée, avec le dérivé organométallique d'un produit de formule (IV) :

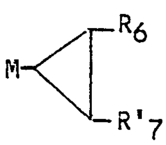

(IV)

dans laquelle M représente un atome de métal alcalin tel que le lithium ou un reste -MgHal, dans lequel Hal représente un atome de chlore, de brome ou d'iode, $R_6$ a la signification déjà indiquée et $R'_7$ représente un atome d'hydrogène ou un radical alcoyle renfermant de 1 à 5 atomes de carbone, pour obtenir un produit de formule (II) :

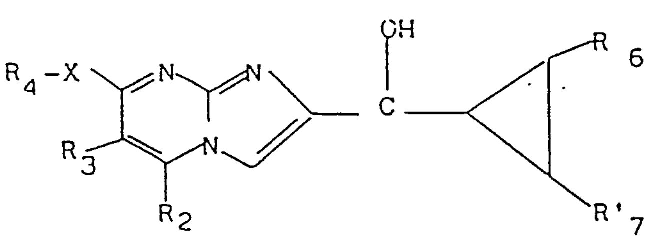

(II)

dans laquelle $R_2$, $R_3$, $R_4$, X, $R_6$ et $R'_7$ ont la signification déjà indiquée, puis oxyde le produit de formule ainsi obtenu, pour obtenir le produit de formule ($I_A$) :

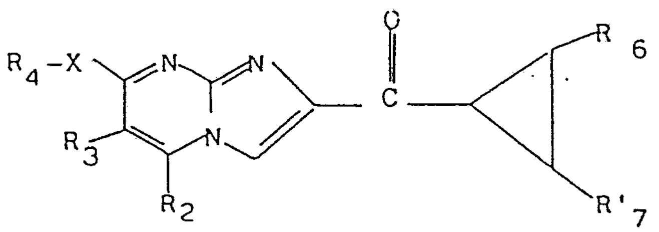

(I_A)

ainsi attendu dans laquelle $R_2$, $R_3$, $R_4$, $R_6$ et $R'_7$ ont la signification déjà indiquée, que l'on peut salifier, le cas échéant.

Dans des conditions préférentielles de mise en oeuvre de l'invention, le procédé de préparation ci-dessus décrit est caractérisé en ce que :

a) la réaction du produit de formule (III) avec le dérivé organométallique d'un produit de formule (IV) est effectuée dans des conditions anhydres, dans un solvent organique tel que le tétrahydrofuranne,

b) l'oxydation du produit de formule (II) est effectuée avec le dioxyde de manganèse, l'acide nitrique, le chlorure ferrique ou l'oxyde de chrome en présence de pyridine ou encore par la méthode d'Oppenauer ou enfin par déshydrogénation en présence d'un catalyseur à base de cuivre.

L'invention a aussi pour objet un procédé de préparation des nouvelles imidazo [1,2-a] pyrimidines telles que définies par la formule (I) ci-dessus ainsi que de leurs sels, caractérisé en ce que l'on fait réagir un produit de formule (III) :

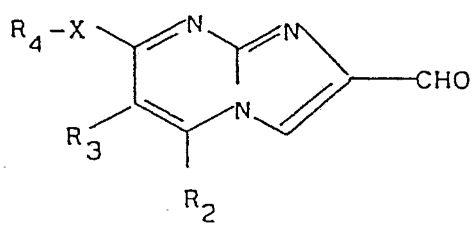

(III)

dans laquelle $R_2$, $R_3$, $R_4$ et X ont la signification déjà indiquée, avec le dérivé organométallique d'un produit de formule (VII) :

$$M-\overset{R_5}{\overset{|}{C}}=CH-R_6$$

(VII)

dans laquelle M représente un atome de métal alcalin tel que le lithium ou un reste -Mg-Hal, dans lequel Hal représente un atome de chlore, de brome ou d'iode, $R_5$ et $R_6$ ont la signification déjà indiquée, pour obtenir un produit de formule (VI) :

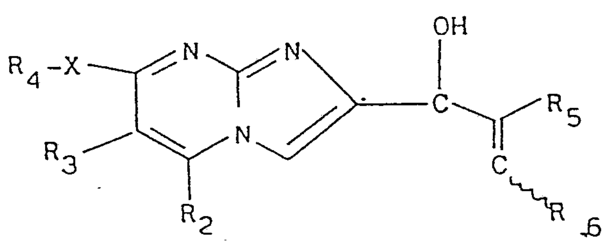

(VI)

dans laquelle $R_2$, $R_3$, $R_4$, $R_5$, $R_6$ et X ont la signification déjà indiquée, puis oxyde le produit de formule (VI) à ainsi obtenu, pour obtenir un produit de formule (V) :

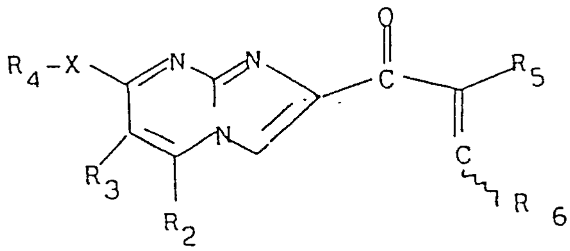

(V)

dans laquelle $R_2$, $R_3$, $R_4$, $R_5$, $R_6$ et X ont la signification déjà indiquée, puis fait réagir ce dernier avec un agent de cyclisation approprié, capable en outre d'introduire le groupe CH-R"$_7$ au niveau de la liaison vinylique, pour obtenir un produit de formule ($I_B$) :

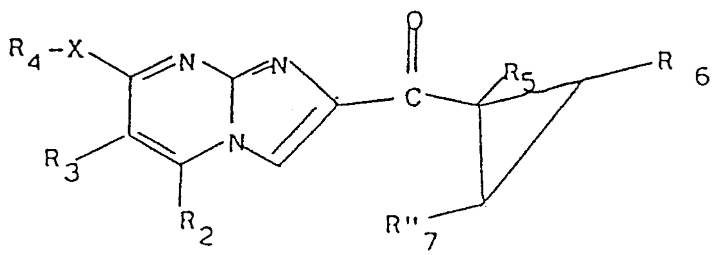

($I_B$)

dans laquelle $R_2$, $R_3$, $R_4$, $R_5$, $R_6$ et X ont la signification déjà indiquée et R"$_7$ représente un atome d'hydrogène, un atome d'halogène, un radical alcoyle renfermant de 1 à 5 atomes de carbone, un radical alcoxy carbonyle renfermant de 2 à 5 atomes de carbone ou un radical phényle, produit de formule ($I_B$) que l'on isole et si désiré salifie ou bien dans le cas où R"$_7$ représente un radical alcoxycarbonyle, que l'on soumet à une hydrolyse pour obtenir un produit de formule ($I_D$) :

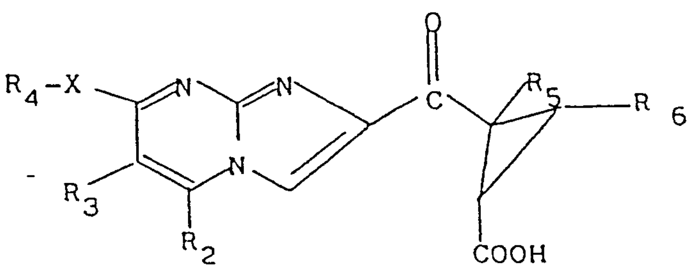

($I_D$)

dans laquelle $R_2$, $R_3$, $R_4$, $R_5$, $R_6$ et X ont la signification déjà indiquée, que l'on isole et si désiré salifie ou bien le cas échéant, fait réagir ledit produit de formule ($I_D$) avec une amine de formule (VIII) :

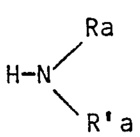

(VIII)

dans laquelle Ra et R'a, identiques ou différents, représente un atome d'hydrogène ou un radical alcoyle renfermant de 1 à 5 atomes de carbone, pour obtenir un produit de formule ($I_C$) :

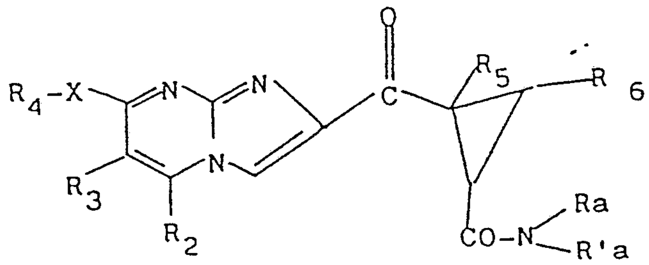

(I$_C$)

dans laquelle $R_2$, $R_3$, $R_4$, $R_5$, $R_6$, Ra, R'a et X ont la signification déjà indiquée, que l'on isole et si désiré salifie ou bien le cas échéant, déshydrate le produit de formule ($I_C$) dans laquelle Ra et R'a représentent un atome d'hydrogène, pour obtenir le produit de formule ($I_E$) :

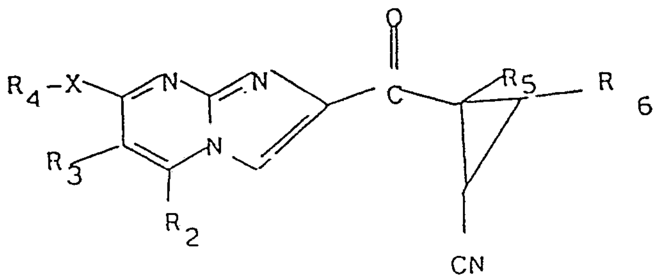

(I$_E$)

recherché dans laquelle $R_2$, $R_3$, $R_4$, $R_5$, $R_6$ et X ont la signification déjà indiquée, que l'on isole et si désiré salifie.

Dans des conditions préférentielles de mise en oeuvre de l'invention, le procédé de préparation ci-dessus décrit est caractérisé en ce que :

a) la réaction du produit de formule (III) avec le dérivé organométallique d'un produit de formule (VII) est effectuée dans des conditions anhydres, dans un solvant organique tel que le tétrahydrofuranne,

b) l'oxydation du produit de formule (VI) est effectuée avec le dioxyde de manganèse, l'acide nitrique, le chlorure ferrique ou l'oxyde de chrome, en présence de pyridine ou encore par la méthode d'Oppenauer ou enfin par déshydrogénation en présence d'un catalyseur à base de cuivre,

c) lorsque l'on désire obtenir un produit de formule ($I_B$) dans laquelle $R_5$ représente un radical alcoyle renfermant de 1 à 5 atomes de carbone, $R_6$ représente un atome d'hydrogène et $R''_7$ représente un atome d'hydrogène ou un radical alcoyle renfermant de 1 à 5 atomes de carbone, la cyclisation est avantageusement effectuée au moyen d'un iodure de trialkyloxosulphonium en opérant au sein d'un solvant organique tel que le diméthylformamide,

d) lorsque l'on désire obtenir un produit de formule ($I_B$) dans laquelle $R_5$ représente un atome d'hydrogène, $R_6$ représente un radical alcoyle renfermant de 1 à 5 atomes de carbone et $R''_7$ représente un atome d'hydrogène ou un radical alcoyle renfermant de 1 à 5 atomes de carbone, la cyclisation est avant ageusement effectuée au moyen d'un tétrafluoroborate de diméthylamidoalkylphényloxosulphonium en opérant au sein d'un solvant organique tel que le diméthylformamide,

e) lorsque l'on désire obtenir un produit de formule ($I_B$) dans laquelle $R_5$ représente un atome d'hydrogène et $R''_7$ représente un groupement alcoxy carbonyle ou un radical phényle, la cyclisation est avantageusement effectuée au moyen d'un acétte d'alcoyle de diméthylsulphuranylidène ou d'un anion de benzyldiméthylsulphonium en opérant au sein d'un solvant organique tel que le chloroforme,

f) lorsque l'on désire obtenir un produit de formule ($I_B$) dans laquelle $R_5$ représente un atome d'hydrogène et $R''_7$ représente un atome d'halogène, la cyclisation est avantageusement effectuée au moyen d'un halogénométhylure de diméthylaminophényloxosulphonium en opérant au sein d'un solvant organique tel que le diméthylformamide,

g) l'hydrolyse du produit de formule ($I_B$) dans laquelle $R''_7$ représente un radical alcoxycarbonyle est effectuée au moyen d'un hydroxyde alcalin tel que l'hydroxyde de sodium,

h) la réaction de produit de formule ($I_D$) avec l'amine de formule (VIII) est effectuée au sein d'un solvant organique anhydre en présence de carbonyldiimidazole,

i) la déshydratation du produit de formule ($I_C$) est effectuée au moyen d'un anhydride d'acide fort tel que l'anhydride d'acide trifluoroacétique au sein d'un solvant organique tel que le dichlorométhane.

Les produits de formule (I) présentent un caractère basique.

On peut avartageusement préparer les sels d'addition des produits de formule (I) en faisant réagir, en proportions sensiblement stoechiométriques, un acide minéral ou organique avec lesdits produits de formule (I). Les sels peuvent être préparés sans isoler les bases correspondantes.

Les produits, objet de la présente invention, possèdent de très intéressantes proprietés pharmacologiques ; ils possèdent notamment une grande affinité pour les récepteurs des benzodiazépines. Certains produits présentent, en outre, des propriétés tranquillisantes.

Ces propriétés sont illustrées plus loin dans la partie expérimentale.

Ces propriétés justifient l'utilisation des nouvelles imidazo [1,2-a] pyrimidines de formule (I), ainsi que de leurs sels, pharmaceutiquement acceptables, à titre de médicaments.

La présente invention a ainsi également pour objet l'application à titre de médicaments des nouvelles imidazo [1,2-a] pyrimidines telles que définies par la formule générale (I) ainsi que de leurs sels d'addition avec les acides pharmaceutiquement acceptables.

Parmi les médicaments, objet de l'invention, on retient notamment les médicaments caractérisés en ce qu'ils sont constitués par les nouvelles imidazo [1,2-a] pyrimidines répondant à la formule (I) dans laquelle $R_2$, $R_3$, $R_4$ et X ont la signification déjà indiquée,
$R_5$ représente un atome d'hydrogène ou un radical méthyle,
$R_6$ représente un atome d'hydrogène ou un radical méthyle,
$R_7$ représente un atome d'hydrogène, un atome de chlore, un radical méthyle, un radical éthoxycarbonyle, un radical cyano, un radical amido, méthylamido ou diméthylamido, étant entendu que les radicaux $R_5$, $R_6$ et $R_7$ ne peuvent pas représenter tous en même temps un atome d'hydrogène ainsi que par leurs sels d'addition avec les acides pharmaceutiquement acceptables.

Parmi les médicaments, objet de l'invention, on retient tout particulièrement ceux caractérisés en ce qu'ils sont constitués par les nouvelles imidazo [1,2-a] pyrimidines répondant à la formule (I), dans laquelle $R_2$ et $R_3$ identiques ou différents, représentent un atome d'hydrogène, un radical méthyle ou éthyle ou $R_2$ et $R_3$ forment ensemble un radical butylène,
X représente un atome d'oxygène ou de soufre,
$R_4$ représente un radical méthyle,
$R_5$, $R_6$ et $R_7$ ont la signification déjà indiquée ainsi que leurs sels d'addition avec les acides pharmaceutiquement acceptables.

Parmi les médicaments préférés de l'invention, on retient tout particulièrement :
- la trans (6,7,8,9-tétrahydro-5-méthoxyimidazo [1,2-a] quinazolin-2-yl 2-chlorocyclopropylméthanone,
- le trans 2-(6-éthyl 7-méthoxy 5-méthylimidazo [1,2-a] pyrimidin-2-oyl) cyclopropane carboxylate d'éthyle,
- le trans 2-(6,7,8,9-tétrahydro-5-méthoxyimidazo [1,2-a] quinazolin-2-oyl) cyclopropane carbonitrile,
- la trans (6-éthyl 7-méthoxy 5-méthylimidazo [1,2-a] pyrimidin-2-yl) 2-méthyl cyclopropylméthanone,
- la (6,7,8,9-tétrahydro 5-méthoxyimidazo [1,2-a] quinazolin-2-yl) 1-méthyl cyclopropylméthanone, ainsi que leurs sels pharmaceutiquement acceptables.

Ces médicaments trouvent, par exemple, leur emploi dans le traitement des troubles de la mémoire, notamment en gériatrie, des troubles de la sénescence cérébrale. Certains produits peuvent également être utilisés dans le traitement de l'obésité ainsi que comme tranquillisants mineurs dans le traitement de certaines agitations ou irratibilité.

La dose usuelle, variable selon le produit utilisé, le sujet traité et l'affection en cause peut être par exemple, de 0,1 mg à 200 mg par jour, par voie orale.

L'invention a également pour objet les compositions pharmaceutiques qui renferment au moins un dérivé précité ou l'un de ses sels d'addition avec les acides pharmaceutiquement acceptables à titre de principe actif.

A titre de médicaments, les dérivés répondant à la formule (I) et leurs sels d'addition avec les acides pharmaceutiquement acceptables peuvent être incorporés dans des compositions pharmaceutiques destinées à la voie digestive ou parentérale.

Ces compositions pharmaceutiques peuvent être, par exemple, solides ou liquides et se présenter sous les formes pharmaceutiques couramment utilisées en médecine humaine, comme par exemple, les comprimés simples ou dragéifiés, les gélules, les capsules, les granulés, les suppositoires, les préparations injectables ; elles sont préparées selon les methodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

L'invention a enfin pour objet, à titre de produits nouveaux et notamment d'intermédiaires nécessaires à la préparation des produits de formule (I) :
- les produits de formule (II) :

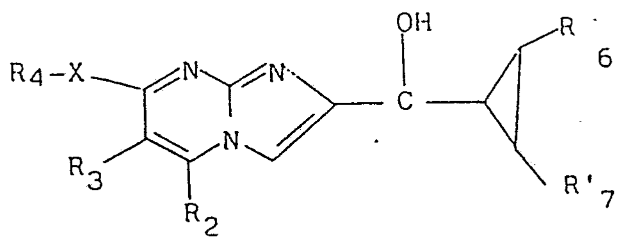

(II)

dans laquelle $R_2$, $R_3$, $R_4$, $R_6$ et X ont la signification déjà indiquée, $R'_7$ représente un atome d'hydrogène ou un radical alcoyle renfermant de 1 à 5 atomes de carbone,
- les produits de formule (VI) :

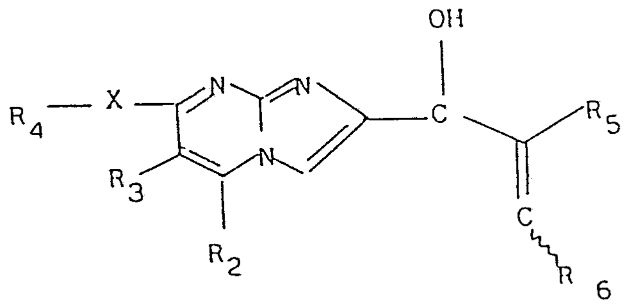

(VI)

dans laquelle $R_2$, $R_3$, $R_4$, $R_5$, $R_6$ et X ont la signification déjà indiquée,
- les produits de formule (V) :

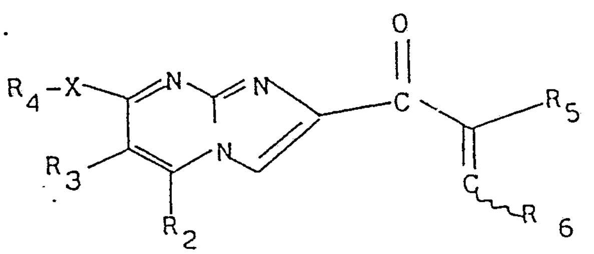

(V)

dans laquelle $R_2$, $R_3$, $R_4$, $R_5$, $R_6$ et X ont la signification déjà indiquée.

Les produits de formule (III) peuvent être préparés comme indiqué dans le brevet britannique n° 2 128 989 B.

Les exemples qui suivent illustrent l'invention sans toutefois la limiter.

**Exemple 1 : trans 2-(6-éthyl 7-méthoxy 5-méthylimidazo [1,2-a] pyrimidin-2-oyl) cyclopropane carboxylate d'éthyle.**

**Stade A** : (6-éthyl 7-méthoxy 5-méthylimidazo [1,2-a] pyrimidin-2-yl) vinyl méthanol.

On ajoute lentement à 0°C sous atmosphère inerte, 55 cm3 d'une solution molaire de bromure de vinyl magnésium dans 8 g de 6-éthyl 7-méthoxy 5-méthylimidazo [1,2-a] pyrimidin-2-carboxaldéhyde en solution dans 300 cm3 de tétrahydrofuranne. On agite 3 heures à température ambiante, arrête la réaction par addition d'une solution aqueuse de chlorure d'ammonium et extrait au chloroforme. On lave la phase organique à l'eau, la sèche et évapore le solvant sous pression réduite. Après purification par chromatographie sur silice (éluant : chloroforme-méthanol), on obtient 6,89 g de produit attendu. F = 118-121°C.

Spectre IR (KBr) :

3140, 2980, 2870, 1635, 1460, 1390, 1260, 1155, 1030 et 990 $cm^{-1}$.

**Stade B** : (6-éthyl 7-méthoxy 5-méthylimidazo [1,2-a] pyrimidin-2-yl) vinyl méthanone.

On ajoute 15 g de dioxyde de manganèse à 6,8 g de produit obtenu au stade A en solution dans 500 cm3 de chloroforme et chauffe 1 heure au reflux. On filtre à chaud le milieu réactionnel sur silice et évapore à sec la phase organique sous pression réduite. On purifie le résidu par chromatographie sur silice (éluant : chloroforme-méthanol) et obtient 3,54 g de produit attendu. F = 151-152°C.

Spectre IR (KBr) :
3140, 3020, 2970, 2960, 2875, 1670, 1640, 1460, 1385, 1315, 1260, 1155, 990, 25 et 770 $cm^{-1}$.

**Stade C** : trans 2-(6-éthyl 7-méthoxy 5-méthylimidazo [1,2-a] pyrimidin-2-oyl) cyclopropane carboxylate d'éthyle.

On ajoute à température ambiante 0,4 g de diméthylsulfuranylidène acétate d'éthyle (préparé selon le procédé décrit dans G. Payne, J. Org. Chem. (1967), 32, (11), 3351) en solution dans 20 cm3 de chloroforme à une solution comprenant 0,5 g de produit préparé au stade B dans 25 cm3 de chloroforme. On agite 2 heures à température ambiante, filtre sur silice en éluant au chloroforme. On évapore sous pression réduite, cristallise le résidu dans le mélange chlorure de méthylène-éther éthylique et obtient 0,63 g de produit attendu.

En opérant comme indiqué aux stades A et B de l'exemple 1, on a préparé les produits suivants :
- (6,7,8,9-tétrahydro 5-méthoxyimidazo [1,2-a] quinazolin-2-yl) vinyl méthanone,
- (6-éthyl 7-méthoxy 5-méthylimidazo [1,2-a] pyrimidin-2-yl) propen-1-yl méthanone,
- (6,7,8,9-tétrahydro 5-méthoxyimidazo [1,2-a] quinazolin-2-yl) propen-2-yl méthanone,
- (6,7,8,9-tétrahydro 5-méthoxyimidazo [1,2-a] quinazolin-2-yl) propen-1-yl méthanone.

En opérant comme indiqué au stade C de l'exemple 1, on a préparé les produits des exemples suivants.

**Exemple 2 : trans 2-(6-éthyl 7-méthoxy 5-méthylimidazo [1,2-a] pyrimidin-2-oyl) 3-méthyl cyclopropane carboxylate d'éthyle.**

**Exemple 3 : trans 2-(6,7,8,9-tétrahydro 5-méthoxyimidazo [1,2-a] quinazolin -2-oyl) 2-méthyl cyclopropane carboxylate d'éthyle.**

**Exemple 4 : (6,7,8,9-tétrahydro 5-méthoxyimidazo [1,2-a] quinazolin-2-yl) 1-méthyl cyclopropyl méthanone.**

On ajoute 5 cm3 d'une solution aqueuse d'hydroxyde de sodium 12,5N dans une suspension comprenant 1 g de (6,7,8,9-tétrahydro 5-méthoxyimidazo [1,2-a] quinazolin-2-yl), 15 g d'iodure de triméthyloxosulphonium et 200 mg de bromure de tétrabutylammonium dans 30 cm3 de chlorure de méthylène. On chauffe 3 heures au reflux, sépare par décantation la phase organique, la lave à l'eau, la sèche, élimine le solvant sous pression réduite puis chromatographie le résidu sur silice (éluant : chlorure de méthylène). On sépare par décantation la phase organique, la lave à l'eau, la sèche et élimine le solvant sous pression réduite. On chromatographie le résidu sur silice (éluant : chlorure de méthylène-acétate d'éthyle 9-1) et obt ient 0,45 g de produit attendu.

**Exemple 5 : trans (6-éthyl 7-méthoxy 5-méthylimidazo [1,2-a] pyrimidin-2- yl) 2-méthyl cyclopropyl méthanone.**

On ajoute 2,2 g de (6-éthyl 7-méthoxy 5-méthylimidazo [1,2-a] pyrimidin-2-yl) propen-1-yl méthanone dans une solution de méthylure de diméthylaminophényloxosulphonium préparée à partir de 2,76 g de tétrafluoroborate de diméthylaminométhylphényloxosulphonium et de 306 mg d'hydrure de sodium à 80% dans 20 cm3 de diméthylformamide selon la méthode de C. Johnson et E. Janiga J. Am. Chem. Soc. (1973), 95, (23), 7692). On agite 1 heure à température ambiante, dilue avec 100 cm3 d'acétate d'éthyle, lave à l'eau, sèche et évapore pour obtenir une huile qui cristallise dans l'éther. On obtient 1,2 g de produit attendu.

**Exemple 6 : trans (6,7,8,9-tétrahydro 5-méthoxyimidazo [1,2 a] quinazolin--yl) 2-chlorocyclopropyl méthanone.**

On ajoute 514 mg de (6,7,8,9-tétrahydro 5-méthoxyimidazo [1,2-a] quinazolin-2-yl) vinyl méthanone dans une solution de chlorométhylure de diméthylaminophényloxosulphonium préparé à partir de 684 mg de diméthylaminochlorométhylphénylsulfoximine obtenu selon le procédé décrit dans C. Johnson et E. Janiga J. Am. Chem. Soc. (1973), 95, 23, 7692) dans 6 cm3 de diméthyl formamide. On agite 30 minutes à température ambiante, dilue avec de l'eau, extrait à l'acétate d'éthyle et obtient après chromatographie, 280 mg de produit attendu.

**Exemple 7 : Acide trans 2-(6,7,8,9-tétrahydro 5-méthoxyimidazo [1,2-a] quinazolin-2-oyl) cyclopropane carboxylique.**

On ajoute 1 g d'hydroxyde de potassium en solution dans 10 cm3 d'eau à 2,88 g de trans 2-(6,7,8,9-tétrahydro 5-méthoxyimidazo [1,2-a] quinazolin-2-oyl) cyclopropane carboxylate d'éthyle obtenu comme à l'exemple 1 à partir du (6,7,8,9-tétrahydro 5-méthoxyimidazo [1,2-a] quinazolin-2-yl) vinyl méthanone en solution dans 40 cm3 de méthanol. On chauffe le mélange à 40°C pendant 2 heures, ajoute 50 cm3 d'eau, élimine le méthanol sous pression réduite. On lave la solution aqueuse au chlorure de méthylène, ajoute de l'acide chlorhydrique 2N jusqu'à l'obtention d'un pH 3. On filtre le précipité, le lave à l'eau, le sèche et récupère 2,1 g de produit attendu.

**Exemple 8 : Acide trans 2-(6-éthyl 7-méthoxy 5-méthylimidazo [1,2-a] pyrimidin-2-oyl) cyclopropane carboxylique.**

On opère comme à l'exemple 7 à partir des produits correspondants et obtient le produit attendu.

**Exemple 9 : trans 2-(6,7,8,9-tétrahydro 5-méthoxyimidazo [1,2-a] quinazolin-2-oyl) cyclopropane carboxamide.**

On ajoute 1,5 g de carbonyldiimidazole à 2 g d'acide trans 2-(6,7,8,9-tétrahydro 5-methoxyimidazo [1,2-a] quinazolin-2-oyl) cyclopropane carboxylique en suspension dans 25 cm3 de diméthylformamide. On chauffe le mélange pendant 3 heures à 50° C, refroidit, soumet à un barbotage d'ammoniac pendant 2 heures et chauffe à nouveau 2 heures à 50° C. On dilue avec 150 cm3 d'eau le milieu réactionnel, filtre et obtient 1,62 g de produit attendu.

**Exemple 10 : trans N,N-diméthyl 2-(6,7,8,9-tétrahydro 5-méthoxyimidazo [1,2-a] quinazolin-2-oyl) cyclopropane carboxamide.**

En opérant comme à l'exemple 9 au départ des produits appropriés, on obtient le produit attendu.

**Exemple 11 : trans 2-(6,7,8,9-tétrahydro 5-méthoxyimidazo [1,2-a] quinazolin-2-oyl) cyclopropane carbonitrile.**

On ajoute lentement 1,5 g d'anhydride trifluoroacétique à 1,6 g de trans 2-(6,7,8,9-tétrahydro 5-méthoxyimidazo [1,2-a] quinazolin-2-oyl) cyclopropane carboxamide en solution dans 25 cm3 de dichlorométhane et agite 15 minutes à température ambiante. On lave àl'eau, élimine le solvant sous pression réduite et cristallise le résidu dans l'éther. On obtient 1,25 g de produit attendu.

Les rendements, microanalyses, points de fusion et analyses spectrométriques des exemples 1, 2, 3, 4, 5, 6, 8 et 11 sont donnés dans le tableau I ci-après.

TABLEAU I

| Ex. | $R_2$ | $R_3$ | $R_5$ | $R_7$ | $R_6$ | Rendt % | F°C | Formule Pds Mol. | Analyse : Calculé Trouvé C% | H% | N% | Spectre IR $cm^{-1}$ |
|---|---|---|---|---|---|---|---|---|---|---|---|---|
| 1 | Me | Et | H | $CO_2Et$ | H | 88 | 169-170 | $C_{17}H_{21}N_3O_4$ 331,376 | 61,62 61,32 | 6,39 6,29 | 12,68 12,54 | 1725, 1660, 1542, 1365, 1300, 1210, 1176 |
| 2 | Me | Et | H | $CO_2Et$ | Me | 60 | 152-153 | $C_{18}H_{23}N_3O_4$ 345,402 | 62,59 62,50 | 6,71 6,72 | 12,17 12,08 | 1723, 1635, 1540, 1360, 1280, 1175 |
| 3 | $-(CH_2)_4-$ | | Me | $CO_2Et$ | H | 65 | 182-184 | $C_{19}H_{23}N_3O_4$ 357,413 | 63,85 63,66 | 6,49 6,45 | 11,76 11,72 | 1725, 1637, 1365, 1250, 1190, 1160 |
| 4 | $-(CH_2)_4-$ | | Me | H | H | 43 | 225-226 | $C_{16}H_{19}N_3O_2$ 285,348 | 67,34 67,04 | 6,71 6,72 | 14,73 14,43 | 3025, 2940, 1635, 1540, 1460, 1368, 1167 |
| 5 | Me | Et | H | Me | H | 52 | 151-153 | $C_{15}H_{19}N_3O_2$ 273,337 | 65,91 65,56 | 7,01 7,00 | 15,37 15,22 | 3120, 1650, 1635, 1540, 1357, 1202, 1179 |
| 6 | $-(CH_2)_4-$ | | H | Cl | H | 40 | 210-212 | $C_{15}H_{16}N_3O_2Cl$ 305,767 | 58,92 58,87 Cl Calc | 5,27 5,31 11,59 | 13,74 13,64 Tr.11,63 | 3130, 2937, 1655, 1638, 1540, 1367, 1170 |
| 8 | Me | Et | H | $CO_2H$ | H | 94 | 220-221 | $C_{15}H_{17}N_3O_4$ 303,320 | 59,40 59,19 | 5,65 5,64 | 13,85 13,82 | 3130, 1665, 1635, 1542, 1304, 1210, 1180 |
| 11 | $-(CH_2)_4-$ | | H | CN | H | 82 | 203-205 | $C_{16}H_{16}N_4O_2$ 296,332 | 64,85 65,00 | 5,44 5,49 | 18,91 18,80 | 3130, 2940, 2240, 1662, 1642, 1545, 1458, 1368, 1200, 1170 |

**Exemple 12 :**

On a préparé des comprimés répondant à la formulation suivante :

- Trans (6,7,8,9-tétrahydro 5-méthoxyimidazo [1,2-a] quinazolin-2-yl) 2-chlorocyclopropyl méthanone 20 mg
- Excipient pour un comprimé terminé à 150 mg.

(Détail de l'excipient : lactose, amidon, talc, stéarate de magnésium).

**Exemple 13 :**

On a préparé des comprimés répondant à la formulation suivante :

- Trans 2-(6-éthyl 7-méthoxy 5-méthylimidazo [1,2-a] pyrimidin-2-oyl) cyclopropane carboxylate d'éthyle 20 mg
- Excipient pour un comprimé terminé à 150 mg.

(Détail de l'excipient : lactose, amidon, talc, stéarate de magnésium).

## ACTIVITE PHARMACOLOGIQUE

L'affinité des composés pour les récepteurs des benzodiazépines a été évaluée en utilisant un radioligang [$^3H$] flunitrazépam et la méthode de Squires et Braestrup (Nature, 1977, 266, 732) modifiée.

Les valeurs indiquées dans le tableau II ci-après sont les concentrations nanomolaires du produit testé qui inhibent dans une proportion de 50% la liaison spécifique de 0,6 nanomoles de [$^3H$] flunitrazépam dans des préparations de membranes de cerveaux antérieurs de rats [($CI_{50}$)en nanomoles].

**TABLEAU II**

| Exemple | Liaison aux récepteurs $CI_{50}$Nm |
|---|---|
| 1 | 560 |
| 4 | 6300 |
| 5 | 1780 |
| 6 | 180 |
| 11 | 1000 |

**Revendications**

1.- Nouvelles imidazo [1,2-a] pyrimidines répondant à la formule (I) :

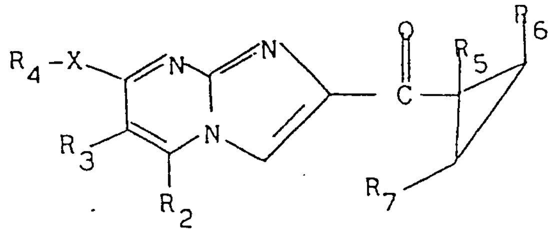

(I)

dans laquelle $R_2$ et $R_3$, identiques ou différents, représentent un atome d'hydrogène, un radical alcoyle linéaire renfermant de 1 à 8 atomes de carbone éventuellement substitué par un radical cycloalcoyle renfermant de 3 à 7 atomes de carbone, un radical alcényle linéaire renfermant de 2 à 5 atomes de carbone, un radical cycloalcoyle renfermant de 3 à 7 atomes de carbone, un radical aralcoyle renfermant de 7 à 15 atomes de carbone ou $R_2$ et $R_3$ forment ensemble un radical alcoylène renfermant de 3 à 5 atomes de carbone, chacun des radicaux ci-dessus pouvant être substitué par un ou plusieurs radicaux alcoyles renfermant de 1 à 5 atomes de carbone,
X représente un atome d'oxygène ou de soufre,
$R_4$ représente un radical alcoyle linéaire renfermant de 1 à 5 atomes de carbone,
$R_5$ représente un atome d'hydrogène ou un radical alcoyle renfermant de 1 à 5 atomes de carbone,
$R_6$ représente un atome d'hydrogène ou un radical alcoyle renfermant de 1 à 5 atomes de carbone,
$R_7$ représente un atome d'hydrogène, un radical alcoyle renfermant de 1 à 5 atomes de carbone, un radical phényle, un atome d'halogène, un radical hydroxycarbonyle, un radical alkoxycarbonyle renfermant de 2 à 5 atomes de carbone, un radical cyano, un radical amido, un radical mono ou dialcoylamido dont les radicaux alcoyles renferment de 1 à 5 atomes de carbone,
étant entendu que l'un au moins des radicaux $R_5$, $R_6$ et $R_7$ ne représentent pas un atome d'hydrogène, ainsi que leurs sels d'addition avec les acides minéraux ou organiques.

2.- Nouvelles imidazo [1,2-a] pyrimidines telles que définies à la revendication 1, ainsi que leurs sels, caractérisés en ce que dans ladite formule (I),
$R_2$, $R_3$, $R_4$ et X ont la signification déjà indiquée,
$R_5$ représente un atome d'hydrogène ou un radical méthyle,
$R_6$ représente un atome d'hydrogène ou un radical méthyle,
$R_7$ représente un atome d'hydrogène, un atome de chlore, un radical méthyle, un radical éthoxycarbonyle, un radical cyano, un radical amido, méthylamido ou diméthylamido,
étant entendu que l'un au moins des radicaux $R_5$, $R_6$ et $R_7$ ne représente pas un atome d'hydrogène.

3.- Nouvelles imidazo [1,2-a] pyrimidines telles que définies à la revendication 1 ou 2, ainsi que leurs sels, caractérisés en ce que dans ladite formule (I) :
$R_2$ et $R_3$, identiques ou différents, représentent un atome d'hydrogène, un radical méthyle ou éthyle, ou $R_2$ et $R_3$ forment ensemble un radical butylène,
X représente un atome d'oxygène ou de soufre,
$R_4$ représente un radical méthyle,
$R_5$, $R_6$ et $R_7$ ont la signification déjà indiquée.

4.- la trans (6,7,8,9-tétrahydro-5-méthoxyimidazo [1,2-a] quinazolin-2-yl 2-chlorocyclopropylméthanone,
- le trans 2-(6-éthyl 7-méthoxy 5-méthylimidazo [1,2-a] pyrimidin-2-oyl) cyclopropane carboxylate d'éthyle,
- le trans 2-(6,7,8,9-tétrahydro-5-méthoxyimidazo [1,2-a] quinazolin-2-oyl) cyclopropane carbonitrile,
- la trans (6-éthyl 7-méthoxy 5-méthylimidazo [1,2-a] pyrimidin-2-yl) 2-méthyl cyclopropylméthanone,
- la (6,7,8,9-tetrahydro 5-méthoxyimidazo [1,2-a] quinazolin-2-yl) 1-méthyl cyclopropylméthanone.

5.- Procédé de préparation des nouvelles imidazo [1,2-a] pyrimidines telles que définies par la formule (I) de la revendication 1, dans laquelle $R_2$, $R_3$, $R_4$, $R_6$ et X ont la signification déjà indiquée, $R_5$ représente un atome d'hydrogène et $R_7$ représente un atome d'hydrogène ou un radical alcoyle renfermant de 1 à 5 atomes de carbone, ainsi que de leurs sels, caractérisé en ce que l'on fait réagir un produit de formule (III) :

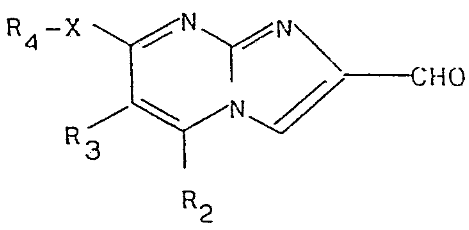

(III)

dans laquelle $R_2$, $R_3$, $R_4$ et X ont la signification déjà indiquée, avec le dérivé organométallique d'un produit de formule (IV) :

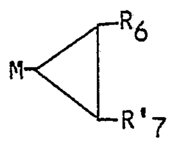

(IV)

dans laquelle M représente un atome de métal alcalin tel que le lithium ou un reste -MgHal, dans lequel Hal représente un atome de chlore, de brome ou d'iode, $R_6$ a la signification déjà indiquée et $R'_7$ représente un atome d'hydrogène ou un radical alcoyle renfermant de 1 à 5 atomes de carbone, pour obtenir un produit de formule (II) :

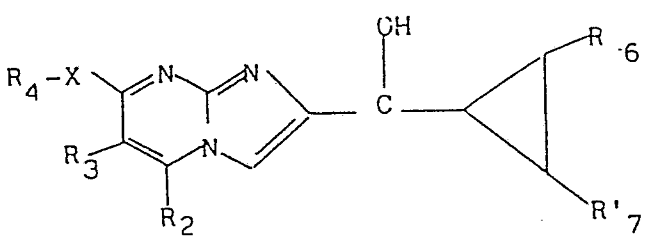

(II)

dans laquelle $R_2$, $R_3$, $R_4$, X, $R_6$ et $R'_7$ ont la signification déjà indiquée, puis oxyde le produit de formule ainsi obtenu, pour obtenir le produit de formule ($I_A$) :

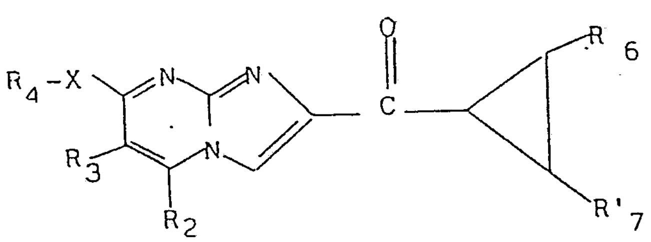

($I_A$)

ainsi attendu dans laquelle $R_2$, $R_3$, $R_4$, $R_6$ et $R'_7$ ont la signification déjà indiquée, que l'on peut salifier, le cas échéant.

6.- Procédé de préparation selon la revendication 5, caractérisé en ce que :

a) la réaction du produit de formule (III) avec le dérivé organométallique d'un produit de formule (IV) est effectuée dans des conditions anhydres, dans un solvant organique tel que le tétrahydrofuranne,

b) l'oxydation du produit de formule (II) est effectuée avec le dioxyde de manganèse, l'acide nitrique, le chlorure ferrique ou l'oxyde de chrome en présence de pyridine ou encore par la méthode d'Oppenauer ou enfin par déshydrogénation en présence d'un catalyseur à base de cuivre.

7.- Procédé de préparation des nouvelles imidazo [1,2-a] pyrimidines telles que définies par la formule (I) de la revendication 1, ainsi que de leurs sels, caractérisé en ce que l'on fait réagir un produit de formule (III) :

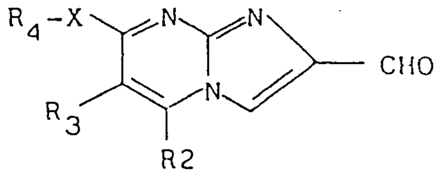

(III)

dans laquelle $R_2$, $R_3$, $R_4$ et X ont la signification déjà indiquée, avec le dérivé organométallique d'un produit de formule (VII) :

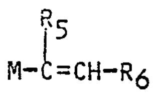

(VII)

dans laquelle M représente un atome de métal alcalin tel que le lithium ou un reste -Mg-Hal, dans lequel Hal représente un atome de chlore, de brome ou d'iode, $R_5$ et $R_6$ ont la signification déjà indiquée, pour obtenir un produit de formule (VI) :

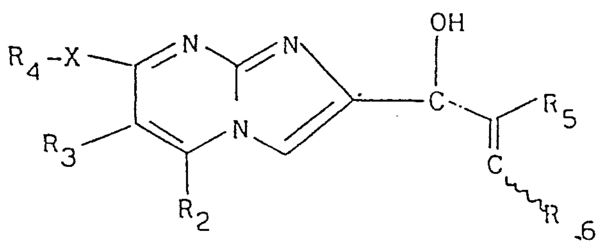

(VI)

dans laquelle $R_2$, $R_3$, $R_4$, $R_5$, $R_6$ et X ont la signification déjà indiquée, puis oxyde le produit de formule (VI) à ainsi obtenu, pour obtenir un produit de formule (V) :

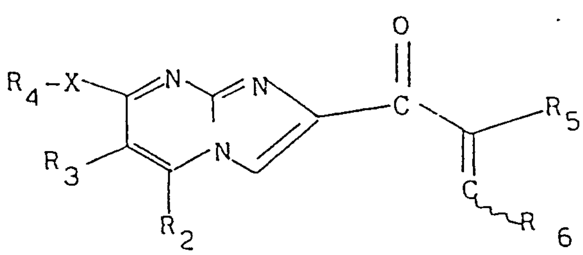

(V)

dans laquelle $R_2$, $R_3$, $R_4$, $R_5$, $R_6$ et X ont la signification déjà indiquée, puis fait réagir ce dernier avec un agent de cyclisation approprié, capable en outre d'introduire le groupe CH-$R''_7$ au niveau de la liaison vinylique, pour obtenir un produit de formule ($I_B$) :

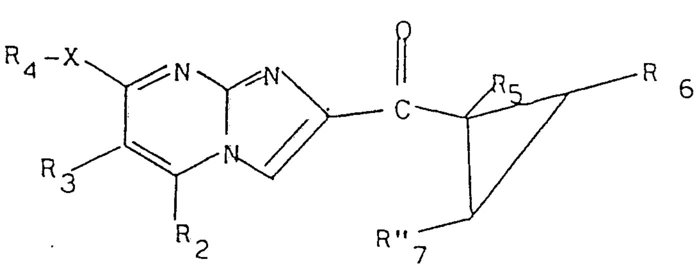

($I_B$)

dans laquelle $R_2$, $R_3$, $R_4$, $R_5$, $R_6$ et X ont la signification déjà indiquée et $R''_7$ représente un atome d'hydrogène, un atome d'halogène, un radical alcoyle renfermant de 1 à 5 atomes de carbone, un radical alcoxy carbonyle renfermant de 2 à 5 atomes de carbone ou un radical phényle, produit de formule ($I_B$)

que l'on isole et si désiré salifie ou bien dans le cas où $R''_7$ représente un radical alcoxycarbonyle, que l'on soumet à une hydrolyse pour obtenir un produit de formule ($I_D$) :

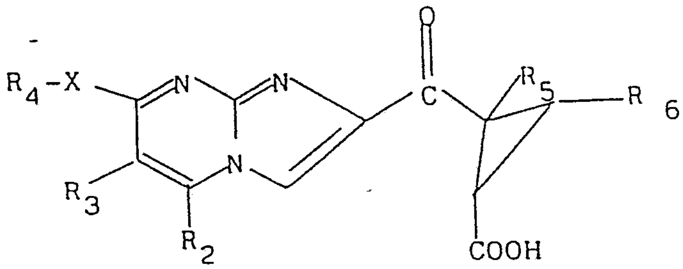

($I_D$)

dans laquelle $R_2$, $R_3$, $R_4$, $R_5$, $R_6$ et X ont la signification déjà indiquée, que l'on isole et si désiré salifie ou bien le cas échéant, fait réagir ledit produit de formule ($I_D$) avec une amine de formule (VIII) :

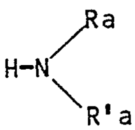

(VIII)

dans laquelle Ra et R'a, identiques ou différents, représente un atome d'hydrogène ou un radical alcoyle renfermant de 1 à 5 atomes de carbone, pour obtenir un produit de formule ($I_C$) :

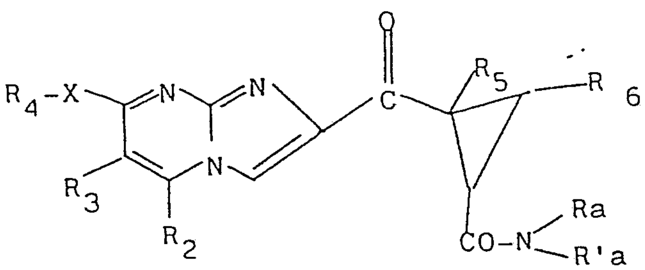

($I_C$)

dans laquelle $R_2$, $R_3$, $R_4$, $R_5$, $R_6$, Ra, R'a et X ont la signification déjà indiquée, que l'on isole et si désiré salifie ou bien le cas échéant, déshydrate le produit de formule ($I_C$) dans laquelle Ra et R'a représentent un atome d'hydrogène, pour obtenir le produit de formule ($I_E$) :

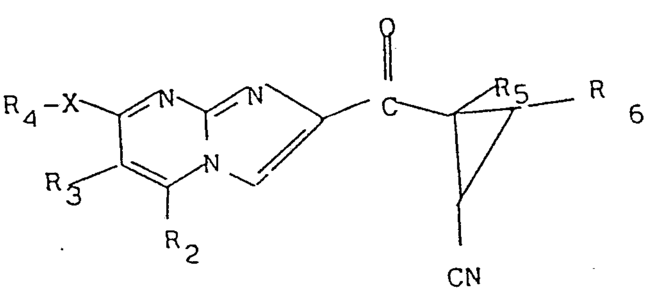

($I_E$)

recherché dans laquelle $R_2$, $R_3$, $R_4$, $R_5$, $R_6$ et X ont la signification déjà indiquée, que l'on isole et si désiré salifie.

8.- Procédé de préparation selon la revendication 7, caractérisé en ce que :

a) la réaction du produit de formule (III) avec le dérivé organométallique d'un produit de formule (VII) est effectuée dans des conditions anhydres, dans un solvant organique tel que le tétrahydrofuranne,

b) l'oxydation du produit de formule (VI) est effectuée avec le dioxyde de manganèse, l'acide nitrique, le chlorure ferrique ou l'oxyde de chrome, en présence de pyridine ou encore par la

méthode d'Oppenauer ou enfin par déshydrogénation en présence d'un catalyseur à base de cuivre,

c) lorsque l'on désire obtenir un produit de formule ($I_B$) dans laquelle $R_5$ représente un radical alcoyle renfermant de 1 à 5 atomes de carbone, $R_6$ représente un atome d'hydrogène et $R''_7$ représente un atome d'hydrogène ou un radical alcoyle renfermant de 1 à 5 atomes de carbone, la cyclisation est avantageusement effectuée au moyen d'un iodure de trialkyloxosulphonium en opérant au sein d'un solvant organique tel que le diméthylformamide,

d) lorsque l'on désire obtenir un produit de formule ($I_B$) dans laquelle $R_5$ représente un atome d'hydrogène, $R_6$ représente un radical alcoyle renfermant de 1 à 5 atomes de carbone et R"7 représente un atome d'hydrogène ou un radical alcoyle renfermant de 1 à 5 atomes de carbone, la cyclisation est avantageusement effectuée au moyen d'un tétrafluoroborate de diméthylaminoalkylphényloxosulphonium en opérant au sein d'un solvant organique tel que le diméthylformamide,

e) lorsque l'on désire obtenir un produit de formule ($I_B$) dans laquelle $R_5$ représente un atome d'hydrogène et $R''_7$ représente un groupement alcoxy carbonyle ou un radical phényle, la cyclisation est avantageusement effectuée au moyen d'un acétate d'alcoyle de diméthylsulphuranylidène ou d'un anion de benzyldiméthylsulphonium en opérant au sein d'un solvant organique tel que le chloroforme,

f) lorsque l'on désire obtenir un produit de formule ($I_B$) dans laquelle $R_5$ représente un atome d'hydrogène et $R''_7$ représente un atome d'halogène, la cyclisation est avantageusement effectuée au moyen d'un halogénométhylure de diméthylaminophényloxosulphonium en opérant au sein d'un solvant organique tel que le diméthylformamide,

g) l'hydrolyse du produit de formule ($I_B$) dans laquelle $R''_7$ représente un radical alcoxycarbonyle est effectuée au moyen d'un hydroxyde alcalin tel que l'hydroxyde de sodium,

h) la réaction de produit de formule ($I_D$) avec l'amine de formule (VIII) est effectuée au sein d'un solvant organique anhydre en présence de carbonyldiimidazole,

i) la déshydratation du produit de formule ($I_C$) est effectuée au moyen d'un anhydride d'acide fort tel que l'anhydride d'acide trifluoroacétique au sein d'un solvant organique tel que le dichlorométhane.

9.- Médicaments, caractérisés en ce qu'ils sont constitués par les nouvelles imidazo [1,2-a] pyrimidines telles que définies par la formule (I) de la revendication 1, ainsi que par leurs sels pharmaceutiquement acceptables.

10.- Médicaments, caractérisés en ce qu'ils sont constitués par les nouvelles imidazo [1,2-a] pyrimidines telles que définies à l'une quelconque des revendications 2, 3 ou 4, ainsi que par leurs sels pharmaceutiquement acceptables.

11.- Compositions pharmaceutiques, caractérisées en ce qu'elles renferment à titre de principe actif, l'un au moins des médicaments, tels que définis à l'une quelconque des revendications 9 ou 10.

12.- A titre de produits industriels nouveaux :

- les produits de formule (II) :

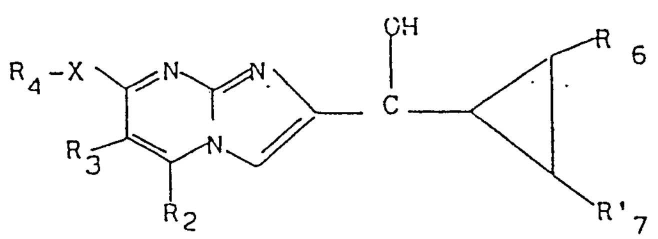

(II)

dans laquelle $R_2$, $R_3$, $R_4$, $R_6$ et X ont la signification déjà indiquée, $R'_7$ représente un atome d'hydrogène ou un radical alcoyle renfermant de 1 à 5 atomes de carbone,

- les produits de formule (VI) :

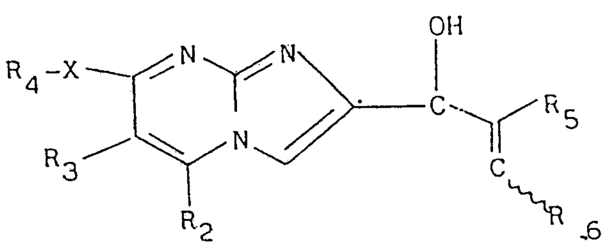

(VI)

dans laquelle $R_2$, $R_3$, $R_4$, $R_5$, $R_6$ et X ont la signification déjà indiquée,

- les produits de formule (V) :

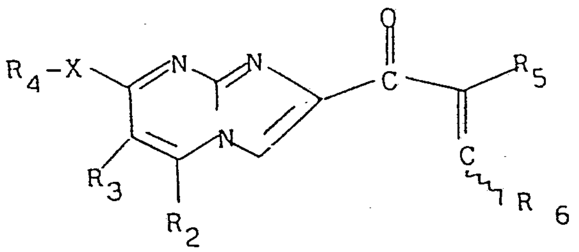

(V)

dans laquelle $R_2$, $R_3$, $R_4$, $R_5$, $R_6$ et X ont la signification déjà indiquée.

**Revendications pour l'Etat contractant suivant: ES**

1.- Procédé de préparation de nouvelles imidazo [1,2-a] pyrimidines répondant à la formule (I) :

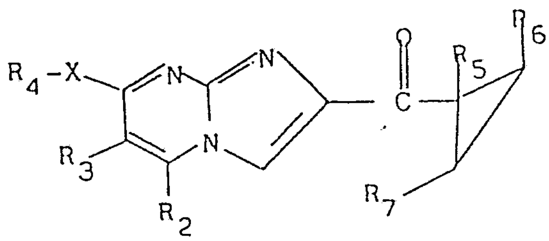

(I)

dans laquelle $R_2$ et $R_3$, identiques ou différents, représentent un atome d'hydrogène, un radical alcoyle linéaire renfermant de 1 à 8 atomes de carbone éventuellement subsitué par un radical cycloalcoyle renfermant de 3 à 7 atomes de carbone, un radical alcényle linéaire renfermant de 2 à 5 atomes de carbone, un radical cycloalcoyle renfermant de 3 à 7 atomes de carbone, un radical aralcoyle renfermant de 7 à 15 atomes de carbone ou $R_2$ et $R_3$ forment ensemble un radical alcoylène renfermant de 3 à 5 atomes de carbone, chacun des radicaux ci-dessus pouvant être substitué par un ou plusieurs radicaux alcoyles renfermant de 1 à 5 atomes de carbone,
X représente un atome d'oxygène ou de soufre,
$R_4$ représente un radical alcoyle linéaire renfermant de 1 à 5 atomes de carbone,
$R_5$ représente un atome d'hydrogène ou un radical alcoyle renfermant de 1 à 5 atomes de carbone,
$R_6$ représente un atome d'hydrogène ou un radical alcoyle renfermant de 1 à 5 atomes de carbone,
$R_7$ représente un atome d'hydrogène, un radical alcoyle renférmant de 1 à 5 atomes de carbone, un radical phényle, un atome d'halogène, un radical hydroxycarbonyle, un radical alkoxycarbonyle renfermant de 2 à 5 atomes de carbone, un radical cyano, un radical amido, un radical mono ou dialcoylamido dont les radicaux alcoyles renferment de 1 à 5 atomes de carbone,
étant entendu que l'un au moins des radicaux $R_5$, $R_6$ et $R_7$ ne représentent pas un atome d'hydrogène, ainsi que leurs sels d'addition avec les acides minéraux ou organiques, caractérisé en ce que l'on fait réagir un produit de formule (III) :

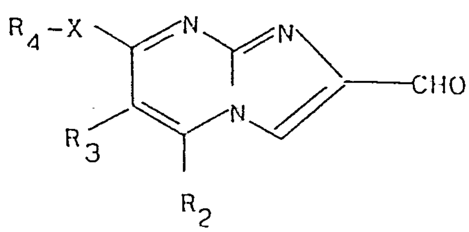

(III)

dans laquelle $R_2$, $R_3$, $R_4$ et X ont la signification déjà indiquée, soit avec le dérivé organométallique d'un produit de formule (IV) :

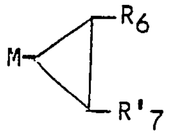

(IV)

dans laquelle M représente un atome de métal alcalin tel que le lithium ou un reste -MgHal, dans lequel Hal représente un atome de chlore, de brome ou d'iode, $R_6$ a la signification déjà indiquée et $R'_7$ représente un atome d'hydrogène ou un radical alcoyle renfermant de 1 à 5 atomes de carbone, pour obtenir un produit de formule (II) :

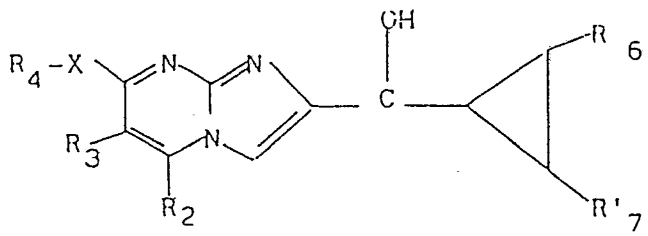

(II)

dans laquelle $R_2$, $R_3$, $R_4$, X, $R_6$ et $R'_7$ ont la signification déjà indiquée, puis oxyde le produit de formule ainsi obtenu, pour obtenir le produit de formule ($I_A$) :

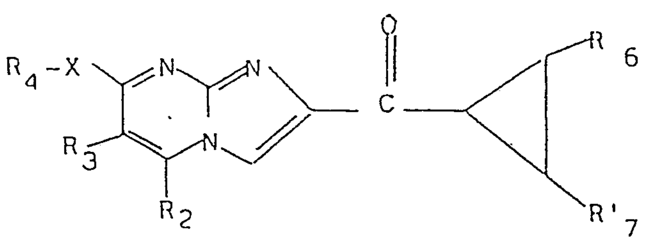

($I_A$)

ainsi attendu dans laquelle $R_2$, $R_3$, $R_4$, $R_6$ et $R'_7$ ont la signification déjà indiquée, correspondant à un produit de formule (I) dans laquelle $R_2$, $R_3$, $R_4$, $R_6$ et X ont la signification déjà indiquée, $R_5$ représente un atome d'hydrogène et $R_7$ représente un atome d'hydrogène ou un radical alcoyle renfermant de 1 à 5 atomes de carbone, produit de formule ($I_A$) que l'on peut salifier, le cas échéant, soit avec le dérivé organométallique d'un produit de formule (VII) :

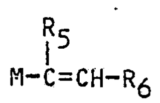

(VII)

dans laquelle M représente un atome de métal alcalin tel que le lithium ou un reste -Mg-Hal, dans lequel Hal représente un atome de chlore, de brome ou d'iode, $R_5$ et $R_6$ ont la signification déjà indiquée, pour obtenir un produit de formule (VI) :

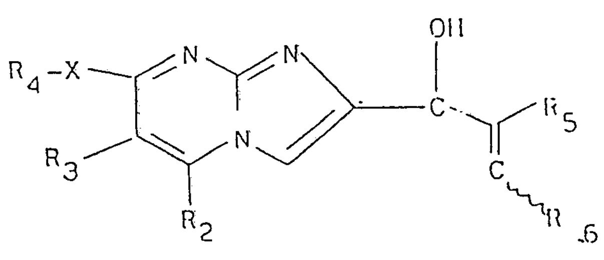

(VI)

dans laquelle $R_2$, $R_3$, $R_4$, $R_5$, $R_6$ et X ont la signification déja indiquée, puis oxyde le produit de formule

(VI)à ainsi obtenu, pour obtenir un produit de formule (V) :

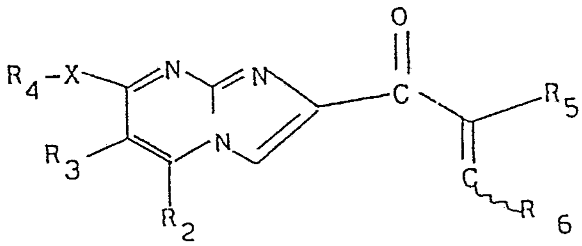

(V)

dans laquelle $R_2$, $R_3$, $R_4$, $R_5$, $R_6$ et X ont la signification déjà indiquée, puis fait réagir ce dernier avec un agent de cyclisation approprié, capable en outre d'introduire le groupe CH-R"$_7$ au niveau de la liaison vinylique, pour obtenir un produit de formule ($I_B$) :

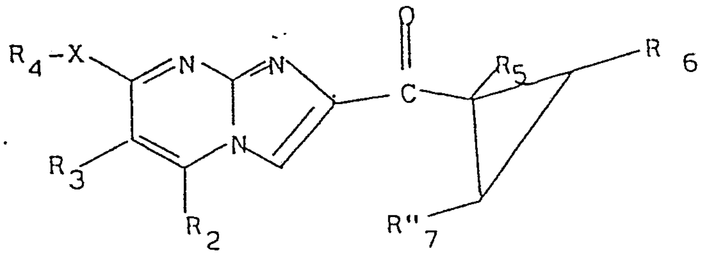

($I_B$)

dans laquelle $R_2$, $R_3$, $R_4$, $R_5$, $R_6$ et X ont la signification déjà indiquée et R"$_7$ représente un atome d'hydrogène, un atome d'halogène, un radical alcoyle renfermant de 1 à 5 atomes de carbone, un radical alcoxy carbonyle renfermant de 2 à 5 atomes de carbone ou un radical phényle, produit de formule ($I_B$) que l'on isole et si désiré salifie ou bien dans le cas où R"$_7$ représente un radical alcoxycarbonyle, que l'on soumet à une hydrolyse pour obtenir un produit de formule ($I_D$) :

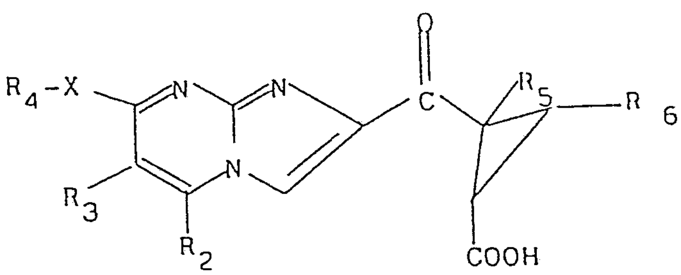

($I_D$)

dans laquelle $R_2$, $R_3$, $R_4$, $R_5$, $R_6$ et X ont la signification déjà indiquée, que l'on isole et si désiré salifie ou bien le cas échéant, fait réagir ledit produit de formule ($I_D$) avec une amine de formule (VIII) :

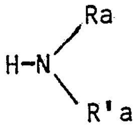

(VIII)

dans laquelle Ra et R'a, identiques ou différents, représente un atome d'hydrogène ou un radical alcoyle renfermant de 1 à 5 atomes de carbone, pour obtenir un produit de formule ($I_C$) :

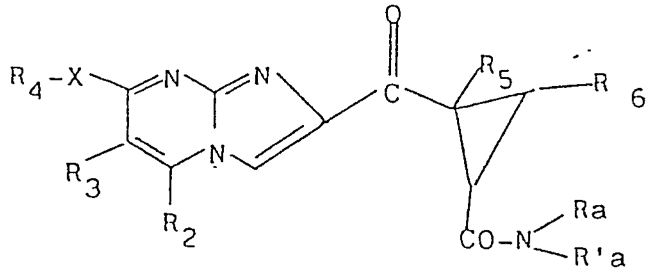

$(I_C)$

dans laquelle $R_2$, $R_3$, $R_4$, $R_5$, $R_6$, Ra, R'a et X ont la signification déjà indiquée, que l'on isole et i désiré salifie ou bien le cas échéant, déshydrate le produit de formule $(I_C)$ dans laquelle Ra et R'a représentent un atome d'hydrogène, pour obtenir le produit de formule $(I_E)$ :

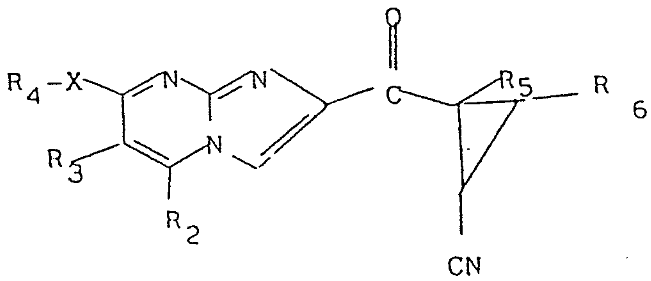

$(I_E)$

recherché dans laquelle $R_2$, $R_3$, $R_4$, $R_5$, $R_6$ et X ont la signification déjà indiquée, que l'on isole et si désiré salifie.

2.- Procédé de préparation selon la revendication 1, caractérisé en ce que :

a) la réaction du produit de formule (III) avec le dérivé organométallique d'un produit de formule (IV) est effectuée dans des conditions anhydres, dans un solvant organique tel que le tétrahydrofuranne,

b) l'oxydation du produit de formule (II) est effectuée avec le dioxyde de manganèse, l'acide nitrique, le chlorure ferrique ou l'oxyde de chrome en présence de pyridine ou encore par la méthode d'Oppenauer ou enfin par déshydrogénation en présence d'un catalyseur à base de cuivre,

c) la réaction du produit de formule (III) avec le dérivé organométallique d'un produit de formule (VII) est effectuée dans des conditions anhydres, dans un solvant organique tel que le tétrahydrofuranne,

d) l'oxydation du produit de formule (VI) est effectuée avec le dioxyde de manganèse, l'acide nitrique, le chlorure ferrique ou l'oxyde de chrome, en présence de pyridine ou encore par la méthode d'Oppenauer ou enfin par déshydrogénation en présence d'un catalyseur à base de cuivre,

e) lorsque l'on désire obtenir un produit de formule $(I_B)$ dans laquelle $R_5$ représente un radical alcoyle renfermant de 1 à 5 atomes de carbone, $R_6$ représente un atome d'hydrogène et $R''_7$ représente un atome d'hydrogène ou un radical alcoyle renfermant de 1 à 5 atomes de carbone, la cyclisation est avantageusement effectuée au moyen d'un iodure de trialkyloxosulphonium en opérant au sein d'un solvant organique tel que le diméthylformamide,

f) lorsque l'on désire obtenir un produit de formule $(I_B)$ dans laquelle $R_5$ représente un atome d'hydrogène, $R_6$ représente un radical alcoyle renfermant de 1 à 5 atomes de carbone et $R''_7$ représente un atome d'hydrogène ou un radical alcoyle renfermant de 1 à 5 atomes de carbone, la cyclisation est avantageusement effectuée au moyen d'un tétrafluoroborate de diméthylaminoalkylphényloxosulphonium en opérant au sein d'un solvant organique tel que le diméthylformamide,

g) lorsque l'on désire obtenir un produit de formule $(I_B)$ dans laquelle $R_5$ représente un atome d'hydrogène et $R''_7$ représente un groupement alcoxy carbonyle ou un radical phényle, la cyclisation est avantageusement effectuée au moyen d'un acétate d'alcoyle de diméthylsulphuranylidène ou d'un anion de benzyldiméthylsulphonium en opérant au sein d'un solvant organique tel que le chloroforme,

h) lorsque l'on désire obtenir un produit de formule $(I_B)$ dans laquelle $R_5$ représente un atome d'hydrogène et $R''_7$ représente un atome d'halogène, la cyclisation est avantageusement effectuée au moyen d'un halogénométhylure de diméthylaminophényloxosulphonium en opérant au sein d'un solvant organique tel que le diméthylformamide,

i) l'hydrolyse du produit de formule $(I_B)$ dans laquelle $R''_7$ représente un radical alcoxycarbonyle est effectuée au moyen d'un hydroxyde alcalin tel que l'hydroxyde de sodium,

j) la réaction de produit de formule ($I_D$) avec l'amine de formule (VIII) est effectuée au sein d'un solvant organique anhydre en présence de carbonyldiimidazole,

k) la déshydratation du produit de formule ($I_C$) est effectuée au moyen d'un anhydride d'acide fort tel que l'anhydride d'acide trifluoroacétique au sein d'un solvant organique tel que le dichlorométhane.

3.- Procédé de préparation selon la revendication 1 ou 2, caractérisé en ce que l'on utilise au départ un produit de formule (IV) dans laquelle $R_6$ représente un atome d'hydrogène ou un radical méthyle et $R'_7$ représente un atome d'hydrogène ou un radical méthyle.

4.- Procédé de préparation selon la revendication 1 ou 2, caractérisé en ce que l'on utilise au départ un produit de formule (VII) dans laquelle $R_5$ représente un atome d'hydrogène ou un radical méthyle, $R_6$ représente un atome d'hydrogène ou un radical méthyle, l'agent de cyclisation que l'on fait réagir sur le produit de formule (V) est choisi de manière tel qu'il puisse introduire le radical $CH-R''_7$ dans lequel $R''_7$ représente un atome d'hydrogène, un atome de chlore, un radical méthyle, un radical éthoxycarbonyle, et que l'on utilise, le cas échéant, un produit de formule (VIII) dans laquelle Ra et R'a, identiques ou différents, représentent un atome d'hydrogène ou un radical méthyle.

5.- Procédé de préparation selon l'une des revendications 1 à 4, caractérisé en ce que l'on utilise au départ un produit de formule (III) dans laquelle $R_2$ et $R_3$, identiques ou différents, représentent un atome d'hydrogène, un radical méthyle ou éthyle, ou $R_2$ et $R_3$ forment ensemble un radical butylène,
X représente un atome d'oxygène ou de soufre,
$R_4$ représente un radical méthyle.

**Revendications pour l'Etat contractant suivant: GR**

1.- Procédé de préparation de nouvelles imidazo [1,2-a] pyrimidines répondant à la formule (I) :

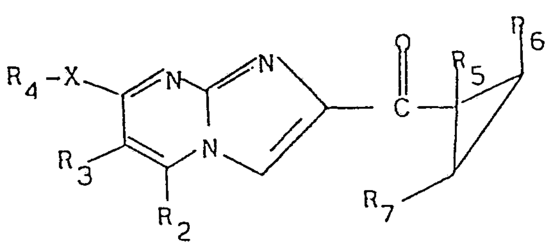

(I)

dans laquelle $R_2$ et $R_3$, identiques ou différents, représentent un atome d'hydrogène, un radical alcoyle linéaire renfermant de 1 à 8 atomes de carbone éventuellement substitué par un radical cycloalcoyle renfermant de 3 à 7 atomes de carbone, un radical alcényle linéaire renfermant de 2 à 5 atomes de carbone, un radical cycloalcoyle renfermant de 3 à 7 atomes de carbone, un radical aralcoyle renfermant de 7 à 15 atomes de carbone ou $R_2$ et $R_3$ forment ensemble un radical alcoylène renfermant de 3 à 5 atomes de carbone, chacun des radicaux ci-dessus pouvant être substitué par un ou plusieurs radicaux alcoyles renfermant de 1 à 5 atomes de carbone,
X représente un atome d'oxygène ou de soufre,
$R_4$ représente un radical alcoyle linéaire renfermant de 1 à 5 atomes de carbone,
$R_5$ représente un atome d'hydrogène ou un radical alcoyle renfermant de 1 à 5 atomes de carbone,
$R_6$ représente un atome d'hydrogène ou un radical alcoyle renfermant de 1 à 5 atomes de carbone,
$R_7$ représente un atome d'hydrogène, un radical alcoyle renfermant de 1 à 5 atomes de carbone, un radical phényle, un atome d'halogène, un radical hydroxycarbonyle, un radical alkoxycarbonyle renfermant de 2 à 5 atomes de carbone, un radical cyano, un radical amido, un radical mono ou dialcoylamido dont les radicaux alcoyles renferment de 1 à 5 atomes de carbone,
étant entendu que l'un au moins des radicaux $R_5$, $R_6$ et $R_7$ ne représentent pas un atome d'hydrogène, ainsi que leurs sels d'addition avec les acides minéraux ou organiques, caractérisé en ce que l'on fait réagir un produit de formule (III) :

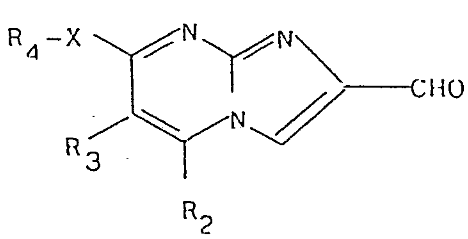

(III)

dans laquelle $R_2$, $R_3$, $R_4$ et X ont la signification déjà indiquée, soit avec le dérivé organométallique d'un

produit de formule (IV) :

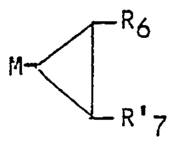

(IV)

dans laquelle M représente un atome de métal alcalin tel que le lithium ou un reste -MgHal, dans lequel Hal représente un atome de chlore, de brome ou d'iode, $R_6$ a la signification déjà indiquée et $R'_7$ représente un atome d'hydrogène ou un radical alcoyle renfermant de 1 à 5 atomes de carbone, pour obtenir un produit de formule (II) :

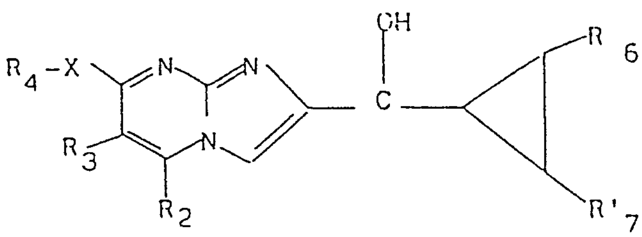

(II)

dans laquelle $R_2$, $R_3$, $R_4$, X, $R_6$ et $R'_7$ ont la signification déjà indiquée, puis oxyde le produit de formule ainsi obtenu, pour obtenir le produit de formule ($I_A$) :

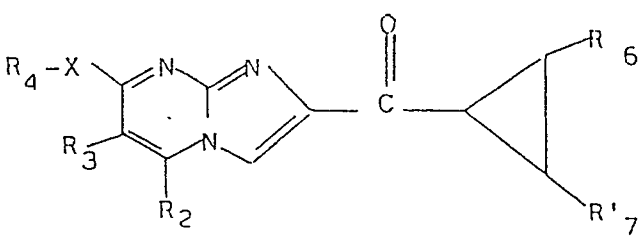

($I_A$)

ainsi attendu dans laquelle $R_2$, $R_3$, $R_4$, $R_6$ et $R'_7$ ont la signification déjà indiquée, correspondant à un produit de formule (I) dans laquelle $R_2$, $R_3$, $R_4$, $R_6$ et X ont la signification déjà indiquée, $R_5$ représente un atome d'hydrogène et $R_7$ représente un atome d'hydrogène ou un radical alcoyle renfermant de 1 à 5 atomes de carbone, produit de formule ($I_A$) que l'on peut salifier, le cas échéant, soit avec le dérivé organométallique d'un produit de formule (VII) :

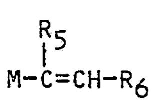

(VII)

dans laquelle M représente un atome de métal alcalin tel que le lithium ou un reste -Mg-Hal, dans lequel Hal représente un atome de chlore, de brome ou d'iode, $R_5$ et $R_6$ ont la signification déjà indiquée, pour obtenir un produit de formule (VI) :

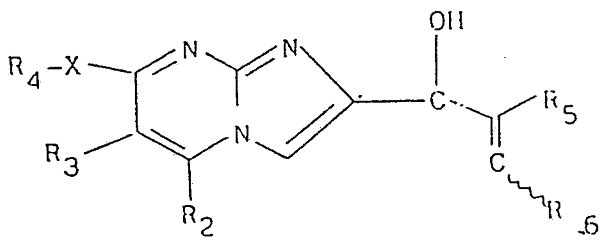

(VI)

dans laquelle $R_2$, $R_3$, $R_4$, $R_5$, $R_6$ et X ont la signification déjà indiquée, puis oxyde le produit de formule (VI)à ainsi obtenu, pour obtenir un produit de formule (V) :

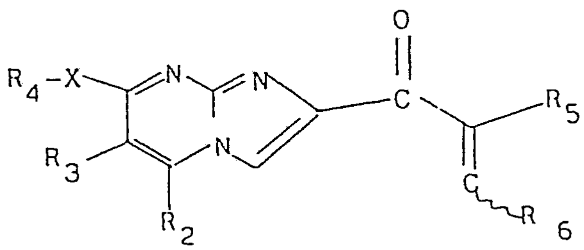

(V)

dans laquelle $R_2$, $R_3$, $R_4$, $R_5$, $R_6$ et X ont la signification déjà indiquée, puis fait réagir ce dernier avec un agent de cyclisation approprié, capable en outre d'introduire le groupe CH-$R''_7$ au niveau de la liaison vinylique, pour obtenir un produit de formule ($I_B$) :

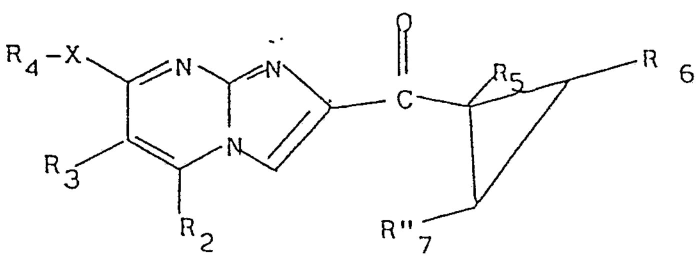

($I_B$)

dans laquelle $R_2$, $R_3$, $R_4$, $R_5$, $R_6$ et X ont la signification déjà indiquée et $R''_7$ représente un atome d'hydrogène, un atome d'halogène, un radical alcoyle renfermant de 1 à 5 atomes de carbone, un radical alcoxy carbonyle renfermant de 2 à 5 atomes de carbone ou un radical phényle, produit de formule ($I_B$) que l'on isole et si désiré salifie ou bien dans le cas où $R''_7$ représente un radical alcoxycarbonyle, que l'on soumet à une hydrolyse pour obtenir un produit de formule ($I_D$) :

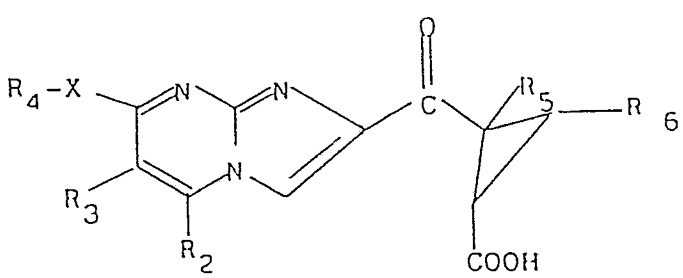

($I_D$)

dans laquelle $R_2$, $R_3$, $R_4$, $R_5$, $R_6$ et X ont la signification déjà indiquée, que l'on isole et si désiré salifie ou bien le cas échéant, fait réagir ledit produit de formule ($I_D$) avec une amine de formule (VIII) :

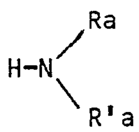

(VIII)

dans laquelle Ra et R'a, identiques ou différents, représente un atome d'hydrogène ou un radical alcoyle renfermant de 1 à 5 atomes de carbone, pour obtenir un produit de formule ($I_C$) :

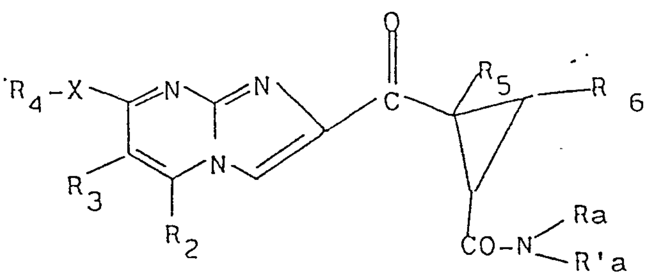

($I_C$)

dans laquelle $R_2$, $R_3$, $R_4$, $R_5$, $R_6$, Ra, R'a et X ont la signification déjà indiquée, que l'on isole et si désiré salifie ou bien le cas échéant, déshydrate le produit de formule ($I_C$) dans laquelle Ra et R'a représentent un atome d'hydrogène, pour obtenir le produit de formule ($I_E$) :

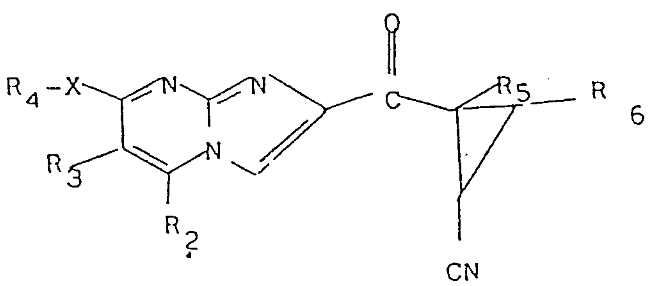

($I_E$)

recherché dans laquelle $R_2$, $R_3$, $R_4$, $R_5$, $R_6$ et X ont la signification déjà indiquée, que l'on isole et si désiré salifie.

2.- Procédé de préparation selon la revendication 1, caractérisé en ce que :

a) la réaction du produit de formule (III) avec le dérivé organométallique d'un produit de formule (IV) est effectuée dans des conditions anhydres, dans un solvant organique tel que le tétrahydrofuranne,

b) l'oxydation du produit de formule (II) est effectuée avec le dioxyde de manganèse, l'acide nitrique, le chlorure ferrique ou l'oxyde de chrome en présence de pyridine ou encore par la méthode d'Oppenauer ou enfin par déshydrogénation en présence d'un catalyseur à base de cuivre,

c) la réaction du produit de formule (III) avec le dérivé organométallique d'un produit de formule (VII) est effectuée dans des conditions anhydres, dans un solvant organique tel que le tétrahydrofuranne,

d) l'oxydation du produit de formule (VI) est effectuée avec le dioxyde de manganèse, l'acide nitrique, le chlorure ferrique ou l'oxyde de chrome, en présence de pyridine ou encore par la méthode d'Oppenauer ou enfin par déshydrogénation en présence d'un catalyseur à base de cuivre,

e) lorsque l'on désire obtenir un produit de formule ($I_B$) dans laquelle $R_5$ représente un radical alcoyle renfermant de 1 à 5 atomes de carbone, $R_6$ représente un atome d'hydrogène et $R''_7$ représente un atome d'hydrogène ou un radical alcoyle renfermant de 1 à 5 atomes de carbone, la cyclisation est avantageusement effectuée au moyen d'un iodure de trialkyloxosulphonium en opérant au sein d'un solvant organique tel que le diméthylformamide,

f) lorsque l'on désire obtenir un produit de formule ($I_B$) dans laquelle $R_5$ représente un atome d'hydrogène, $R_6$ représente un radical alcoyle renfermant de 1 à 5 atomes de carbone et $R''_7$ représente un atome d'hydrogène ou un radical alcoyle renfermant de 1 à 5 atomes de carbone, la cyclisation est avantageusement effectuée au moyen d'un tetrafluoroborate de diméthylaminoalkylphényloxosulphonium en opérant au sein d'un solvant organique tel que le diméthylformamide,

g) lorsque l'on désire obtenir un produit de formule ($I_B$) dans laquelle $R_5$ représente un atome

d'hydrogène et $R''_7$ représente un groupement alcoxy carbonyle ou un radical phényle, la cyclisation est avantageusement effectuée au moyen d'un acétate d'alcoyle de diméthylsulphuranylidène ou d'un anion de benzyldiméthylsulphonium en opérant au sein d'un solvant organique tel que le chloroforme,

h) lorsque l'on désire obtenir un produit de formule ($I_B$) dans laquelle $R_5$ représente un atome d'hydrogène et $R''_7$ représente un atome d'halogène, la cyclisation est avantageusement effectuée au moyen d'un halogénométhylure de diméthylaminophényloxosulphonium en opérant au sein d'un solvant organique tel que le diméthylformamide,

i) l'hydrolyse du produit de formule ($I_B$) dans laquelle $R''_7$ représente un radical alcoxycarbonyle est effectuée au moyen d'un hydroxyde alcalin tel que l'hydroxyde de sodium,

j) la réaction de produit de formule ($I_D$) avec l'amine de formule (VIII) est effectuée au sein d'un solvant organique anhydre en présence de carbonyldiimidazole,

k) la déshydratation du produit de formule ($I_C$) est effectuée au moyen d'un anhydride d'acide fort tel que l'anhydride d'acide trifluoroacétique au sein d'un solvant organique tel que le dichlorométhane.

3.- Procédé de préparation selon la revendication 1 ou 2, caractérisé en ce que l'on utilise au départ un produit de formule (IV) dans laquelle $R_6$ représente un atome d'hydrogène ou un radical méthyle et $R'_7$ représente un atome d'hydrogène ou un radical méthyle.

4.- Procédé de préparation selon la revendication 1 ou 2, caractérisé en ce que l'on utilise au départ un produit de formule (VII) dans laquelle $R_5$ représente un atome d'hydrogène ou un radical méthyle, $R_6$ représente un atome d'hydrogène ou un radical méthyle, l'agent de cyclisation que l'on fait réagir sur le produit de formule (V) est choisi de manière tel qu'il puisse introduire le radical CH-$R''_7$ dans lequel $R''_7$ représente un atome d'hydrogène, un atome de chlore, un radical méthyle, un radical éthoxycarbonyle, et que l'on utilise, le cas échéant, un produit de formule (VIII) dans laquelle Ra et R'a, identiques ou différents, représentent un atome d'hydrogène ou un radical méthyle.

5.- Procédé de préparation selon l'une des revendications 1 à 4, caractérisé en ce que l'on utilise au départ un produit de formule (III) dans laquelle $R_2$ et $R_3$, identiques ou différents, représentent un atome d'hydrogène, un radical méthyle ou éthyle, ou $R_2$ et $R_3$ forment ensemble un radical butylène,
X représente un atome d'oxygène ou de soufre,
$R_4$ représente un radical méthyle.

6.- Procédé de préparation selon l'une des revendications 1 à 5, caractérisé en ce que l'on prépare l'un des produits dont les noms suivent :
- la trans (6,7,8,9-tétrahydro-5-méthoxyimidazo [1,2-a] quinazolin-2-yl 2-chlorocyclopropylméthanone,
- le trans 2-(6-éthyl 7-méthoxy 5-méthylimidazo [1,2-a] pyrimidin-2-oyl) cyclopropane carboxylate d'éthyle,
- le trans 2-(6,7,8,9-tétrahydro-5-methoxyimidazo [1,2-a] quinazolin-2-oyl) cyclopropane carbonitrile,
- la trans (6-éthyl 7-méthoxy 5-méthylimidazo [1,2-a] pyrimidin-2-yl) 2-méthyl cyclopropylméthanone,
- la (6,7,8,9-tétrahydro 5-méthoxyimidazo [1,2-a] quinazolin-2-yl) 1-méthyl cyclopropylméthanone,
ainsi que leurs sels d'addition avec les acides minéraux ou organiques.

7.- A titre de produits industriels nouveaux,
- les produits de formule (II) :

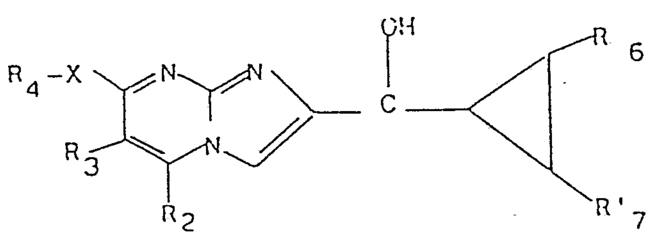

(II)

dans laquelle $R_2$, $R_3$, $R_4$, $R_6$ et X ont la signification déjà indiquée, $R'_7$ représente un atome d'hydrogène ou un radical alcoyle renfermant de 1 à 5 atomes de carbone,
- les produits de formule (VI) :

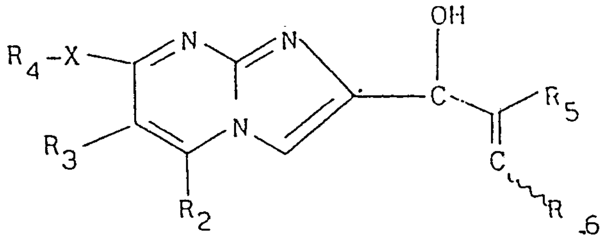

(VI)

dans laquelle $R_2$, $R_3$, $R_4$, $R_5$, $R_6$ et X ont la signification dé]a indiquée,
- les produits de formule (V) :

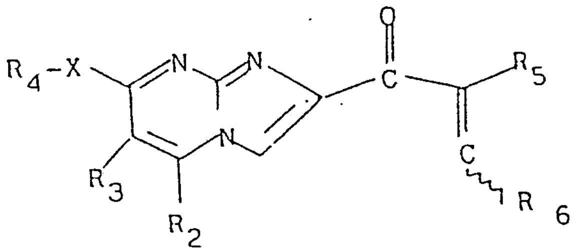

(V)

dans laquelle $R_2$, $R_3$, $R_4$, $R_5$, $R_6$ et X ont la signification déjà indiquée.

8.- Procédé de préparation de compositions pharmaceutiques, caractérisé en ce que l'on met à titre de principe actif, l'un au moins des composés de formule (I), tel qu'obtenu à la revendication 1 ou l'un au moins de ses sels pharmaceutiquement acceptables, sous une forme destinée à cet emploi.

9.- Procédé de préparation de compositions pharmaceutiques, caractérisé en ce que l'on met à titre de principe actif, l'un au moins des composés de formule (I), tel qu'obtenu à l'une quelconque des revendications 2 à 5 ou l'un au moins de ses sels pharmaceutiquement acceptables, sous une forme destinée à cet emploi.

10.- Procédé de préparation de compositions pharmaceutiques, caractérisé en ce que l'on met à titre de principe actif, l'un au moins des composés de formule (I), tel qu'obtenu à la revendication 5 ou l'un au moins de ses sels pharmaceutiquement acceptables, sous une forme destinée à cet emploi.